# EUROPÄISCHE PATENTSCHRIFT

(11) **EP 2 170 791 B1**
(45) Veröffentlichungstag und Bekanntmachung des Hinweises auf die Patenterteilung: **08.05.2013**
(21) Anmeldenummer: 08759035.2
(22) Anmeldetag: 05.06.2008
(51) Int. Cl.: C07C 67/14, C07C 29/159, C07C 67/08, C12P 7/62, C12P 41/00

(54) **VERFAHREN ZUR HERSTELLUNG VON CYCLOPENT-4-EN-1,3-DIOL ODER CYCLOPENT-4-EN-1,3-DIOL-DERIVATEN**
METHOD FOR PRODUCING CYCLOPENT-4-ENE-1,3-DIOL OR CYCLOPENT-4-ENE-1,3-DIOL DERIVATIVES
PROCEDE DE PRODUCTION DE CYCLOPENT-4-ENE-1,3-DIOL OU DE DERIVES DE CYCLOPENT-4-ENE-1,3-DIOL

(30) Priorität: 13.06.2007 DE 102007027189
(43) Veröffentlichungstag der Anmeldung: 07.04.2010
(73) Patentinhaber: Euticals GmbH, 65929 Frankfurt am Main (DE)
(72) Erfinder: JUNG, Jörg, 65439 Flörsheim (DE); LEHNEMANN, Bernd, Wilhelm, 51063 Köln (DE)
(74) Vertreter: Schweitzer, Klaus
(86) Internationale Anmeldenummer: PCT/EP2008/004482
(87) Internationale Veröffentlichungsnummer: WO 2008/151750

(56) Entgegenhaltungen:
- EP-A- 1 428 888
- WO-A-96/30320
- US-A- 5 728 899
- T.CURRAN ET AL: "The preparation of optically active 2-cyclopenten-1,4-diol derivatives from furfuryl alcohol" TETRAHEDRON, Bd. 53, Nr. 6, 1997, Seiten 1983-2004, XP004105280 in der Anmeldung erwähnt

## Beschreibung

Optisch aktive Cyclopenten-1,4-diol-Derivate sind vielfältig verwendbare Ausgangsprodukte für die Synthese von Prostaglandinen, carbocyclischen Nucleosiden und anderen biologisch aktiven Produkten, die durch enzymatische partielle Verseifung von Estern des cis-Cyclopent-4-en-1,3-diols oder durch enzymatische partielle Veresterung von cis-Cyclopent-4-en-1,3-diol in enantiomerenangereicherter Form zugänglich sind (siehe z. B. EP 1 428 888 A1 oder Tetrahedron Letters 1984, 25 (51), 5875-78). All diese Verfahren haben jedoch gemeinsam, daß als Ausgangsprodukt entweder cis-Cyclopent-4-en-1,3-diol oder ein geeigneter Ester (meist das Diacetat) verwendet wird.

Für diese Vorprodukte sind unterschiedliche Herstellwege bekannt, die jedoch sämtlich mit Nachteilen behaftet sind, die ihre Übertragung in den technischen Maßstab verhindern, wie weiter unten beschrieben.

Eine bekannter Herstellweg besteht in der Epoxidierung von Cyclopentadien mittels Persäuren oder Peroxiden, gefolgt von Kupfer(I)-katalysierter Umlagerung (Marino et al., Tetrahedron Lett. 1983, 24, 5, 441-444) oder gefolgt von einer Palladium(0)-katalysierten Umlagerung (Organic Synthesis 1993, Coll. Vol 8, Seite 13). Die Handhabung von organischen Peroxiden im technischen Maßstab stellt jedoch ein untragbares Sicherheitsrisiko dar und verbietet sich daher. Das gleiche gilt für die Handhabung von Cyclopentadien, das bereits bei Temperaturen deutlich unter Raumtemperatur in einer stark exothermen Reaktion zu dimerisieren beginnt und daher nur weit unter Raumtemperatur und sehr verdünnt gelagert bzw. gehandhabt werden kann. Besondere Probleme für die technische Umsetzung ergeben sich aus der Kombination beider Sicherheitsrisiken, die die Anwendung des Verfahrens in der Technik praktisch ausschließt.

Eine weitere Variante besteht in der Reaktion von Cyclopentadien mit photochemisch erzeugtem Singulett-Sauerstoff und Reduktion des entstehenden Addukts mit Thioharnstoff (Johnson et al., J. Am. Chem. Soc. 1986, 108, 18, 5655-5656). Diese Variante ist ebenfalls aus technischen Gründen zur Herstellung größerer Mengen Produkt wenig geeignet.

Eine weitere Variante geht von preiswertem Furfurylalkohol, aus nachwachsenden Rohstoffen gewonnen, aus, der bei leicht saurem pH-Wert zu 4-Hydroxycyclopent-2-enon umlagert. Dieses kann nun - gegebenenfalls gereinigt und mit geschützer Hydroxyfunktion - durch selektive Reduktion des Enons in cis-Cyclopent-4-en-1,3-diol bzw. dessen Derivate umgesetzt werden (Curran et al., Tetrahedron 1997, 53, 6, 1983-2004). Diese Reduktion verläuft häufig mit unzureichender Selektivität, so daß das Produkt mit größeren Mengen unerwünschter trans-Verbindungen und gesättigter Cyclopentanderivate (d.h. Produkte der 1,4-Reduktion) verunreinigt ist, die technisch nicht oder nur schwierig abtrennbar sind. Nachteilig ist zudem, daß das Ausgangsmaterial der Reduktion zunächst nur mit 40 bis 60%iger Reinheit anfällt und aufgrund seiner thermischen Empfindlichkeit nur schwer destillativ gereinigt werden kann. Eine geeignete Reduktionsmethode müßte daher in der Lage sein, direkt das Rohprodukt mit hoher Selektivität zu reduzieren.

Die besten Ergebnisse werden durch Reduktion mit Aluminium- und Bor-Hydriden erzielt. Aluminiumhydride haben jedoch den Nachteil, daß die Hydroxy-Funktion des Ausgangsmaterials 4-Hydroxycyclopent-2-enon vor der Reduktion geschützt und nach der Reduktion wieder entschützt werden muß, wozu die für die enzymatische Verseifung benötigte Alkanoat-Gruppe i. A. ungeeignet ist, da sie durch das Aluminiumhydrid ebenfalls reduziert wird. Daraus resultiert eine lange Sequenz von Reaktionsschritten (Umlagerung von Furfurol - Schützen - Reduktion - Entschützen - Acylierung - enzymatische Verseifung) mit hohen Kosten und geringen Gesamtausbeuten.

Bei der Verwendung von Borhydriden erzielt nur die sogenannte Luche-Reduktion (Curran et al., Tetrahedron 1997, 53, 6, 1983-2004) zufriedenstellende Selektivitäten, die den eklatanten Nachteil hat, in Gegenwart eines größeren Überschusses (1 bis 2 Äquivalente) von Cer(III)-Salzen (im allgemeinen in Form ihrer Hydrate) zu arbeiten. So werden etwa für die Reduktion von 10 g 4-Hydroxycyclopent-2-enon unter klassischen "Luche-Bedingungen" 38 bis 76 g Cer(III)-chlorid-Heptahydrat benötigt. Abgesehen von den hohen resultierenden Kosten für Rohstoffe und Abfallentsorgung, gestaltet sich bei diesen Mengenverhältnissen natürlich auch die Abtrennung der Salze im Zuge der Reaktionsaufarbeitung sehr schwierig, insbesondere da das Reaktionsprodukt Cyclopent-4-en-1,3-diol sehr hydrophil ist, was eine einfache wäßrige Aufarbeitung mit Abtrennung der Salze in der Wasserphase unmöglich macht.

Daher ist keines der bekannten Verfahren geeignet, Cyclopent-4-en-1,3-diol oder Derivate wie beispielsweise Ester im kommerziellen, technischen Maßstab zu akzeptablen Kosten herzustellen.

Aufgabe der vorliegenden Erfindung war es, ein Verfahren zur Verfügung zu stellen, welches geeignet ist, Cyclopent-4-en-1,3-diol oder Derivate wie organische Diester aus dem preisgünstig durch Umlagerung von Furfurol zugänglichen 4-Hydroxycyclopent-2-enon herzustellen, wobei
- keine Schutzgruppenoperationen notwendig sind,
- keine großen Mengen von Cer(III)-Salzen benötigt werden,
- keine großen Mengen von Schwermetall-Abfällen anfallen,
- wenige betriebliche Operationen benötigt werden,
- ungereinigtes Ausgangsmaterial eingesetzt werden kann und
- gute Ausbeuten bei hoher Selektivität zugunsten des ungesättigten cis-Produkts erzielt werden.

Die vorliegende Erfindung löst diese Aufgabe und betrifft ein Verfahren zur Herstellung von cis-Cyclopent-4-en-1,3-diol und cis-Cyclopent-4-en-1,3-dialkanoaten durch selektive cis-1,2-Reduktion von 4-Hydroxycyclopent-2-enon **(I)** mittels eines Borhydrids **(II)** in Gegenwart von substöchiometrischen Mengen einer dreiwertigen Seltenerdmetall-Verbindung **(III)** zu Cyclopent-4-en-1,3-diol **(IV),** welches dann optional zur Vereinfachung der Aufarbeitung und mit oder bevorzugt ohne Zwischenisolierung mit einem Acylierungsmittel **(V)** zu cis-Cyclopent-4-en-1,3-dialkanoaten **(VI)** umgesetzt werden kann. Dabei kann ungereinigtes Roh-Ausgangsmaterial eingesetzt werden und alle Operationen können in einem Ein-Topf-Verfahren durchgeführt werden:

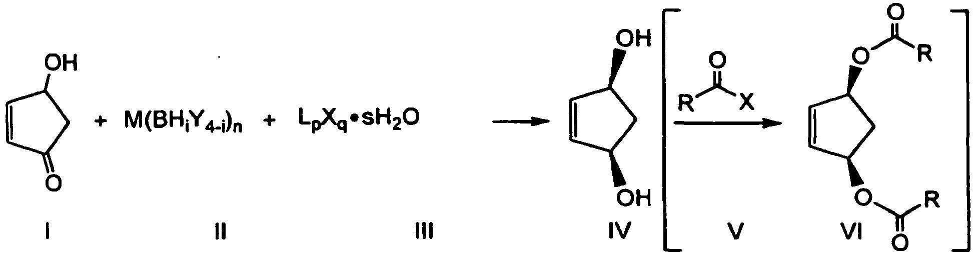

Dabei steht
- M: für ein Alkalimetall, ein Erdalkalimetall, für Zink oder Zirkonium,
- i: für eine ganze Zahl zwischen 1 und 4,
- n: für eine ganze Zahl zwischen 1 und 4 entsprechend der Wertigkeit von M
- Y: für einwertige Anionen, die typischerweise für die Modifizierung von Borhydriden verwendet werden, wie beispielsweise Cyanid oder Acetat,
- L: steht für jedes dreiwertige Seltenerdmetall (oder für Mischungen aus dreiwertigen Seltenerdmetallen),
- X: für ein beliebiges Anion organischer oder anorganischer Säuren,
- p und q: sind unabhängig voneinander ganze Zahlen zwischen 1 und 4 entsprechend der Wertigkeit von X und dem Seltenerdmetall,
- s: ist eine beliebige Zahl zwischen 0 und 20, die den Wassergehalt des verwendeten Seltenerdmetall-Salzes wiedergibt (die Wassergehalte vieler Seltenerdmetall-Salze sind unstöchiometrisch).

Das Acylierungsmittel **V** beschreibt ein System aus einer Verbindung zur Übertragung der Acylgruppe R-CO, optional enthaltend eine zum Abfangen einer evtl. entstehenden Säure zugesetzten Base und gegebenenfalls enthaltend einen Acylierungskatalysator.
- R: ist ein aromatischer oder aliphatischer Rest.

Unter einem aromatischen Rest wird erfindungsgemäß ein zyklisches Molekül mit mindestens einem Ring verstanden, in dem alle Atome *sp*²-hybridisiert sind und welches bevorzugt (4n+2)π-Elektronen aufweist. Besonders bevorzugt handelt es sich hierbei um C₅-C₁₀-aryle, worin ein oder zwei Kohlenstoffatome durch Heteroatome wie N, S oder O ersetzt sein können. Beispiele für solche Reste sind: Phenyl, Naphthyl, Pyridyl, Chinolyl, Pyrimidyl, Chinazolyl, Furyl, Benzofuryl, Pyrrolyl, Indolyl, Thiophenyl, Benzothiophenyl, Imidazolyl, Benzimidazolyl, Pyrazolyl, Indazolyl, Oxazolyl, Benzoxazolyl, Isoxazolyl, Benzisoxazolyl, Thiazolyl, Benzthiazolyl. Bevorzugt sind Phenyl, Naphthyl, Pyridyl, Furyl und Thiophenyl.

Unter einem aliphatischen Rest wird erfindungsgemäß ein C₁-C₁₈-, bevorzugt C₁-C₈-, besonders bevorzugt C₁-C₄-Alkylrest verstanden, der im Falle >C₂ geradkettig, verzweigt oder auch zyklisch sein kann. Ganz besonders bevorzugt ist Methyl, Ethyl und Propyl.

Sofern nichts anderes gesagt ist, handelt es sich bei "%" um "Gew.-%".

I kann als Rohprodukt eingesetzt werden, Gehalte von >40 % sind ausreichend, bevorzugt sind Gehalte von >50 %.

**II** ist ein komplexes Borhydrid wie Calciumborhydrid oder Zinkborhydrid, bevorzugt ein Alkalimetall-Borhydrid wie Natriumborhydrid oder Kaliumborhydrid, besonders bevorzugt Natriumborhydrid.

III ist bevorzugt ein Cer(III)-Halogenid oder ein Halogenid anderer dreiwertiger Seltenerdmetalle oder eine Mischung von dreiwertigen Seltenerdmetallen, besonders bevorzugt Cer(III)-chlorid mit einem Wassergehalt < 1 % oder einem Wassergehalt zwischen 1 % und 40 %, insbesondere Cer(III)-chlorid-Heptahydrat.

Bezogen auf Verbindung I beträgt die notwendige Menge an Seltenerdverbindung weniger als 100 mol%, insbesondere weniger als 50 mol%, bevorzugt weniger als 30 mol%, besonders bevorzugt weniger als 15 mol%.

V besteht bevorzugt aus einem Alkansäureanhydrid oder Alkanoylhalogenid oder einer Alkansäure und einem wasserentziehenden Reagenz wie Propanphosphonsäureanhydrid, Isobutylchlorformiat oder Pivaloylchlorid, besonders bevorzugt Acetylchlorid oder Acetanhydrid, in Verbindung mit einer Base, bevorzugt Triethylamin oder Pyridin. Der optional zugesetzte Katalysator ist bevorzugt ein hypernucleophiler Acylierungskatalysator, besonders bevorzugt 4-Dimethylaminopyridin.

Die Reduktion wird zweckmäßigerweise in einem Lösungsmittel oder Lösungsmittelgemisch, enthaltend Methanol und gegebenenfalls Wasser, Tetrahydrofuran, 2-Methyltetrahydrofuran, tert-Butylmethylether, Diisopropylether, Dipropylether, Dibutylether, 1,4-Dioxan, Toluol, Xylol, Hexan, Heptan oder Petrolether, durchgeführt. Besonders bevorzugt wird die Reduktion in Methanol oder Gemischen von Methanol und einem Ether durchgeführt, wobei dieser Ether besonders bevorzugt der Gruppe {Tetrahydrofuran, 2-Methyltetrahydrofuran, 1,4-Dioxan} entstammt. Im Falle von Gemischen sind solche mit weniger als 50 % Methanol bevorzugt, besonders bevorzugt solche mit weniger als 25 %.

Die optionale Acylierung zur Vereinfachung der Aufarbeitung wird zweckmäßigerweise entweder in einem Gemisch von Methanol und einem Ether, bevorzugt entstammend der Gruppe {Tetrahydrofuran, 2-Methyltetrahydrofuran, 1,4-Dioxan, tert-Butylmethylether, Diisopropylether} oder einem aprotischen Lösungsmittel, bevorzugt entstammend der Gruppe {Alkane, Arene, Ester, Ketone, Ether, N,N-Dimethylformamid, Dimethylsulfoxid, N-Methylpyrrolidon), oder einem Gemisch von Methanol mit mehreren solcher Lösungsmittel durchgeführt. Besonders bevorzugte Lösungsmittel bzw. -gemische enthalten neben Methanol Tetrahydrofuran, 2-Methyltetrahydrofuran, 1,4-Dioxan, tert-Butylmethylether, Ethylacetat, Butylacetat, Isobutylmethylketon, Toluol, Xylol.

Die Reduktion wird zwischen -100 °C und +50 °C durchgeführt, bevorzugt zwischen -100 °C und 0 °C, besonders bevorzugt bei -80 bis -50 °C. Dabei führt die Reaktion im bevorzugten und besonders bevorzugten Temperaturbereich zu Produkten mit höherer Stereoselektivität.

Die Acylierung wird bevorzugt zwischen -20 °C und Raumtemperatur durchgeführt, ganz besonders bevorzugt bei -10 bis +10 °C.

Die Reduktion und die Acylierung können in dem gleichen Lösungsmittel durchgeführt werden. In einer bevorzugten Ausführungsform wird die Reduktion in Methanol durchgeführt, dieses dann abdestilliert und durch ein höhersiedendes aprotisches Lösungsmittel ersetzt, in dem die Acylierung durchgeführt wird (Lösungsmitteltausch).

In einer weiteren bevorzugten Ausführungsform wird die Reduktion einem Gemisch von Methanol und einen aprotischen Lösungsmittel (s.o.) durchgeführt.

Die Acylierung wird zweckmäßigerweise im gleichen Lösemittelgemisch durchgeführt, wobei bevorzugt ein Überschuß von Acylierungsmittel eingesetzt wird, um den Methanolanteil des Lösungsmittels in den entsprechende Methylester umzuwandeln.

Es ist auch bereits möglich, ausgefallene Seltenerdmetall-Salze vor der Acylierung durch Filtration abzutrennen. Ist dies nicht möglich, sollte die Menge des Acylierungsmittel so erhöht werden, daß restliches Kristallwasser durch Acylierungsmittel verbraucht wird.

Die Reaktion kann so durchgeführt werden, daß die Seltenerdmetall-Verbindung zusammen mit dem Ausgangsprodukt **I** in einem Lösungsmittel oder Lösungsmittelgemisch (gemäß obiger Definition) vorgelegt wird und dann portionsweise mit dem Borhydrid **II** versetzt wird. Die bevorzugte Dosiergeschwindigkeit bzw. Portionsgröße wird so gewählt, daß möglichst wenig Überreduktion (1,4-Reduktion) des Substrats eintritt.

Ein bevorzugter Durchführungsmodus der Reduktion besteht aber darin, das Ausgangsmaterial (ggf. in einem Lösungsmittel oder Lösungsmittelgemisch gemäß obiger Definition) und das Borhydrid (ggf. in einem Lösungsmittel oder Lösungsmittelgemisch gemäß obiger Definition) portions- und wechselweise zur vorgelegten Lösung oder Suspension der Seltenerdmetall-Verbindung (in Lösungsmittel oder Lösungsmittelgemisch gemäß obiger Definition) zu dosieren, wobei der jeweilige Reaktand bevorzugt dann zudosiert wird, wenn die jeweils vorher zugegebene Menge desselben Reaktanden größtenteils verbraucht worden ist. Die Größe der einzelnen Portionen des Ausgangsmaterials liegt bevorzugt unterhalb der verwendeten Stoffmenge an Seltenerdmetall-Verbindung.

Ein weiterer bevorzugter Durchführungsmodus der Reduktion besteht darin, eine Lösung oder Suspension von Substrat und Seltenerdmetall-Verbindung (ggf. in einem Lösungsmittel oder Lösungsmittelgemisch gemäß obiger Definition) kontinuierlich mit dem Borhydrid (ggf. in einem Lösungsmittel oder Lösungsmittelgemisch gemäß obiger Definition) umzusetzen.

Die Ausbeuten dieser Reaktion an (cis)-**IV** bzw. (cis)-**VI** bezogen auf die Einsatzmenge an **I** liegen üblicherweise bei >30 %, bevorzugt >50 % und besonders bevorzugt >70 %. Das Verhältnis von (cis)-**IV** bzw. (cis)-**VI** zu (trans)-**IV** bzw. (trans)-**VI** ist erfindungsgemäß besser als 7: 1, bevorzugt besser als 10 : 1, besonders bevorzugt besser als 15 : 1, wobei bei geeigneter Reaktionsführung (Temperatur bei der Reduktion zwischen -50 °C und -100 °C) typischerweise ein Verhältnis von (cis)-**IV** bzw. (cis)-**VI** zu (trans)-**IV** bzw. (trans)-**VI** von ca. 20 : 1 erreicht wird. Der Anteil der Nebenprodukte, die durch "Überreduktion" (1,4-Reduktion gefolgt von 1,2-Reduktion) entstehen, liegt bei Durchführung der Reduktion zwischen 0 °C und Raumtemperatur nicht höher als 20 mol% (bezogen auf Verbindung **I),** bei Durchführung zwischen -100 °C und -50 °C nicht über 10 %.

Das beschriebene Verfahren bietet erstmals einen technisch und wirtschaftlich realisierbaren Herstellweg für Derivate des Cyclopent-4-en-1,3-diols und belegt erstmalig die Durchführbarkeit einer Luche-Reduktion mit substöchiometrischen Mengen an Seltenerdmetall-Verbindung. Es soll durch die folgenden Beispiele erläutert werden:

### Beispiele

### Beispiel 1 (Fahrweise in Methanol/THF mit alternierender Zugabe bei 0 °C):

4,2 g Cer(III)-chlorid Heptahydrat (11,2 mmol) werden bei Raumtemperatur in 6,3 g Methanol gelöst. Dann werden 27,2 g Tetrahydrofuran zugesetzt und auf 0 bis 5 °C gekühlt. Nun wird bei dieser Temperatur mit genau einem Zehntel einer Lösung von 5,0 g 4-Hydroxycyclopent-2-enon (ca. 50 %ig) in 5,0 g THF versetzt und 10 min nachgerührt. Zu diesem Gemisch wird nun wiederum bei 0 bis 5 °C auf einmal genau ein Zehntel von 0,64 g Natriumborhydrid (17,0 mmol) zugesetzt und 20 min nachgerührt. Anschließend wird das nächste Zehntel Edukt-Lösung zugegeben, 10 min gerührt, das nächste Zehntel Natriumborhydrid zugegeben und 20 min nachgerührt. Dieser Zyklus wird solange wiederholt, bis die gesamte Eduktlösung und das gesamte Natriumborhydrid zugesetzt ist. Es wird nun noch 30 min nachgerührt und dann mit 41,3 g Triethylamin versetzt. Zu der entstehenden Lösung/Suspension werden nun bei 0 bis 5 °C 0,25 g 4-Dimethylaminopyridin (2,1 mmol) als Katalysator zugesetzt und dann wird innerhalb von ca. 30.Min bei 0 bis 5 °C mit 41,6 g Essigsäureanhydrid (407,7 mmol) versetzt und es wird noch 120 min bei dieser Temperatur gerührt, um den Umsatz zu vervollständigen. Anschließend wird zunächst mit 42,0 ml Methyl-t-butylether und dann mit 42,00 ml Wasser versetzt. Nun werden die Phasen getrennt und die organische Phase wird soweit wie möglich einrotiert. Nach Destillation bei 0 bis 2 mbar über eine Mikrodestille bei 80 bis 90 °C @ 1 mbar werden so 3,9 g Produkt erhalten, das gemäß GC-Analytik ca. 80 % des gewünschten Produktes, 8 % des Trans-Isomeren und insgesamt 10 % c1s4mns-1,3-Diacetoxycyclopentan (VII) enthält (Ausbeute: 42 % ohne Berücksichtigung der Reinheiten von Edukt und Produkt und ca. 66 % nach Korrektur um Gehalt Edukt und Gehalt Produkt)

### Beispiel 2 (Fahrweise in Methanol/THF mit alternierender Zugabe bei -65 °C):

Wie Beispiel 1, jedoch alternierende Zugabe von Borhydrid und Edukt-Lösung bei -60 bis -70 °C. Die Ausbeute an Rohprodukt bleibt bei dieser Fahrweise nahezu unverändert (4,0 g), jedoch ist die Selektivität der Reaktion deutlich erhöht und damit die Reinheit des Produktes signifikant besser. Dies gilt sowohl für das Verhältnis von cis- zu trans -Cyclopent-4-en-1,3-diacetat (20 : 1) als auch für den Anteil an überreduzierten Verbindungen im Produkt (*cis*/*trans*-1,3-Diacetoxycyclopentan, 2 %).

### Beispiel 3 (Fahrweise in Methanol bei 0 °C, Zugabe Natriumborhydrid):

8,36 g Cer(III)-chlorid Heptahydrat werden in 25,1 g Methanol gelöst und dann werden 10,0 g 4-Hydroxycyclopent-2-enon (ca. 50 %ig) zugesetzt. Anschließend wird auf 0 °C gekühlt und mit einem Zehntel von insgesamt 1,54 g Natriumborhydrid (40,8 mmol) versetzt und ca. 30 min nachgerührt. Dann wird mit einem weiteren Zehntel Natriumborhydrid versetzt, wieder 30 min nachgerührt und in dieser Weise fortgefahren, bis das gesamte Borhydrid zugeben ist. Anschließend wird über Nacht bei RT nachgerührt und am nächsten Tag wird mit 3,9 g Aceton (67,3 mmol) versetzt, um überschüssiges Natriumborhydrid zu zerstören. Das resultierende Gemisch wird am Rotationsverdampfer vom Lösungsmittel so weit wie möglich befreit, mit 50,0 g THF versetzt und noch einmal einrotiert, um soviel Methanol wie möglich zu entfernen. Anschließend wird wieder mit 50,0 g THF versetzt und dann mit 30,9 g Triethylamin und 0,5 g 4-Dimethylaminopyridin (4,1 mmol) als Katalysator. Zu diesem Gemisch werden nun bei 0 bis 10 °C 31,2 g Essigsäureanhydrid (305,8 mmol) zugetropft und es wird noch 60 min nachgerührt, um die Umsetzung zu vervollständigen. Dann wird mit 50 ml Methyl-tert-butylether und langsam mit 31,0 g Wasser versetzt. Nach Phasentrennung wird die organische Phase am Rotationsverdampfer so weit wie möglich vom Lösungsmittel befreit und der resultierende ölige Rückstand wird über ein Mikrodestille destilliert. Es fallen 8,4 g eines ca. 75 %igen Produktes an, das neben dem erwünschten cis -Cyclopent-4-en-1,3-diacetat noch ca. 8 % cis -Cyclopent-4-en-1,3-diacetat und 15 % überreduzierte Verunreinigungen enthält. Ausbeute: 44,7 % ohne Berücksichtigung der Gehalte von Edukt und Produkt und ca. 66 % nach Korrektur um Gehalt Edukt und Gehalt Produkt.

### Beispiel 4 (Fahrweise in Methanol bei -65 °C, Zugabe Natriumborhydrid):

Wie Beispiel 3, jedoch Zugabe von Borhydrid bei -60 bis -70 °C. Die Ausbeute an Rohprodukt bleibt bei dieser Fahrweise wieder nahezu unverändert (8,2 g), jedoch ist die Selektivität der Reaktion deutlich höher und damit die Reinheit des Produktes verbessert. Dies gilt sowohl für das Verhältnis von cis- zu trans -Cyclopent-4-en-1,3-diacetat (20 : 1) als auch für den Anteil an überreduzierten Verbindungen im Produkt (*cis*/*trans*-1,3-Diacetoxycyclopentan, 5 %).

### Beispiel 5 (Fahrweise in Methanol bei -65 °C, alternierende Zugabe)

8,36 g Cer(III)-chlorid Heptahydrat (0,22 Äq.) werden in 25,1 g Methanol gelöst und auf -65 °C gekühlt. Zu diesem Gemisch werden nun genau ein Zehntel einer Mischung aus 10,0 g 4-Hydroxycyclopent-2-enon (ca. 50 %ig) und 5,0 g Methanol zugesetzt und 10 min nachgerührt. Dann wird bei dieser Temperatur mit genau einem Zehntel von insgesamt 1,54 g Natriumborhydrid (40,8 mmol) versetzt und ca. 20 min nachgerührt. Die Zugabe von Eduktlösung und Natriumborhydrid werden in gleicher Weise so lange fortgeführt, bis alles zugegeben ist. Anschließend wird über Nacht bei -65 °C nachgerührt und am nächsten Tag wird mit 3,9 g Aceton (67,3 mmol) versetzt, um überschüssiges Natriumborhydrid zu zerstören. Das resultierende Gemisch wird am Rotationsverdampfer vom Lösungsmittel so weit wie möglich befreit, mit 50,0 g THF versetzt und noch einmal einrotiert, um soviel Methanol wie möglich zu entfemen. Anschließend wird wieder mit 50,0 g THF versetzt und dann mit 30,9 g Triethylamin und 0,5 g 4-Dimethylaminopyridin (4,1 mmol) als Katalysator. Zu diesem Gemisch werden nun bei 0 bis 10 °C 31,2 g Essigsäureanhydrid (305,8 mmol) zugetropft und es wird noch 60 min nachgerührt, um die Umsetzung zu vervollständigen. Dann wird mit 50 ml Methyl-tert-butylether und langsam mit 31,0 g Wasser versetzt. Nach Phasentrennung wird die organische Phase am Rotationsverdampfer so weit wie möglich vom Lösungsmittel befreit und der resultierende ölige Rückstand über ein Mikrodestille destilliert. Es fallen 8,6 g eines ca. 90 %igen Produktes an, das neben dem erwünschten cis -Cyclopent-4-en-1,3-diacetat noch ca. 4 % cis -Cyclopent-4-en-1,3-diacetat und 2 % überreduzierte Verunreinigungen enthält. Ausbeute: 45,8 % bezogen auf Edukt tel quel und ca. 82,4 % nach Korrektur um Gehalt Edukt (ca. 50 %ig) und Gehalt Produkt.

## Patentansprüche

1. Verfahren zur Herstellung von cis-Cyclopent-4-en-1,3-diol aus 4-Hydroxycyclopent-2-enon durch selektive Reduktion mittels komplexer Borhydride in Gegenwart von dreiwertigen Seltenerdmetall-Verbindungen gemäß folgender Gleichung 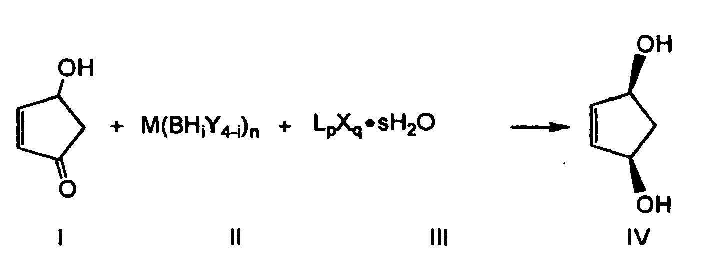 wobei
M für ein Alkalimetall, ein Erdalkalimetall, für Zink oder Zirkonium,
i für eine ganze Zahl zwischen 1 und 4,
n für eine ganze Zahl zwischen 1 und 4 entsprechend der Wertigkeit von M
Y für ein einwertiges Anion,
L für ein dreiwertige Seltenerdmetall (oder für Mischungen aus dreiwertigen Seltenerdmetallen),
X für ein Anion einer organischen oder anorganischen Säure steht,
p und q unabhängig voneinander ganze Zahlen zwischen 1 und 4 sind, entsprechend der Wertigkeit von X und dem Seltenerdmetall,
s eine beliebige Zahl zwischen 0 und 20 ist, die den Wassergehalt des verwendeten Seltenerdmetall-Salzes wiedergibt und
wobei die Seltenerdmetallverbindung, bezogen auf das eingesetzte 4-Hydroxycyclopent-2-enon, in substöchiometrischer Menge eingesetzt wird.

2. Verfahren zur Herstellung von cis-Cyclopent-4-en-1,3-dioldialkanoaten aus 4-Hydroxycyclopent-2-enon durch selektive Reduktion mittels komplexer Borhydride in Gegenwart von dreiwertigen Seltenerdmetall-Verbindungen gefolgt von einer Acetylierung mit oder ohne Isolierung des intermediär gebildeten cis-Cyclopent-4-en-1,3-diols gemäß folgender Gleichung: 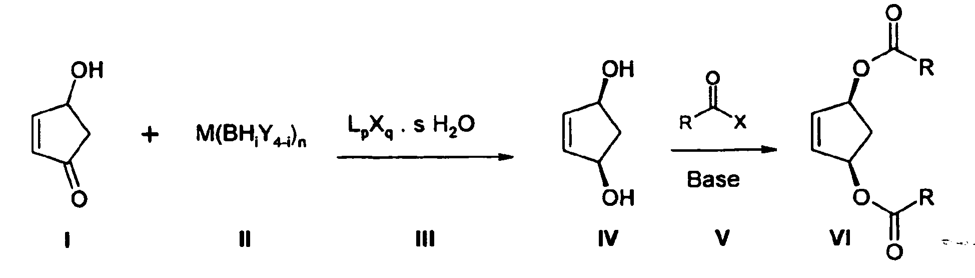 wobei M, i, Y, n, L, X, p, q und s die in Anspruch 1 angegebene Bedeutung haben und
R ein aromatischer oder aliphatischer Rest ist und
wobei die Seltenerdmetallverbindung, bezogen auf das eingesetzte 4-Hydroxycyclopent-2-enon, in substöchiometrischer Menge eingesetzt wird.

3. Verfahren nach Anspruch 1 oder 2, **dadurch gekennzeichnet, daß** weniger als 50 mol% Seltenerdmetall-Verbindung oder Misch-Seltenerdmetall-Verbindung bezogen auf 4-Hydroxycyclopent-2-enon eingesetzt werden.

4. Verfahren nach einem der Ansprüche 1 bis 3, **dadurch gekennzeichnet, daß** das dreiwertige Seltenerdmetall Cer ist.

5. Verfahren nach einem der Ansprüche 1 bis 4, **dadurch gekennzeichnet, daß** das dreiwertige Seltenerdmetallsalz hydratisiertes Cer(III)-chlorid mit einem Wassergehalt zwischen 1 % und 40 % ist.

6. Verfahren nach einem der Ansprüche 1 bis 4, **dadurch gekennzeichnet, daß** das dreiwertigen Seltenerdmetallsalz trockenes Cer(III)-chlorid mit einem Wassergehalt <1 % ist.

7. Verfahren nach einem der Ansprüche 1 bis 6, **dadurch gekennzeichnet, daß** das komplexe Borhydrid Natriumborhydrid, Kaliumborhydrid, Calciumborhydrid oder Zinkborhydrid ist.

8. Verfahren nach einem der Ansprüche 1 bis 7, **dadurch gekennzeichnet, daß** der Reduktionsschritt in einem Lösungsmittel oder Lösungsmittelgemisch, enthaltend Methanol und gegebenenfalls Wasser, Tetrahydrofuran, 2-Methyltetrahydrofuran, tert-Butylmethylether, Düsopropylether, Dipropylether, Dibutylether, 1,4-Dioxan, Toluol, Xylol, Hexan, Heptan oder Petrolether, durchgeführt wird.

9. Verfahren nach einem der Ansprüche 2 bis 8, **dadurch gekennzeichnet, daß** der Acylierungsschritt im gleichen Lösungsmittel oder Lösungsmittelgemisch wie die Reduktion durchgeführt wird.

10. Verfahren nach einem der Ansprüche 2 bis 9, **dadurch gekennzeichnet, daß** zwischen Reduktion und Acylierung ein Lösungsmitteltausch stattfindet und die Acylierung in einem aprotischen Lösungsmittel durchgeführt wird.

11. Verfahren nach einem der Ansprüche 2 bis 10, **dadurch gekennzeichnet, daß** zur Acylierung Essigsäureanhydrid oder Acetylchlorid in Gegenwart einer Base verwendet werden.

12. Verfahren nach einem der Ansprüche 2 bis 11, **dadurch gekennzeichnet, daß** das Acylierungsmittel durch Aktivierung einer Carbonsäure mit Hilfe eines wasserentziehenden Aktivierungsmittels in situ erzeugt wird.

13. Verfahren nach einem der Ansprüche 1 bis 12, **dadurch gekennzeichnet, daß** das Produkt in einer enzymatisch vermittelten Reaktion zu einem nichtracemischen Monoester verseift wird.

14. Verfahren nach einem der Ansprüche 1 bis 13, **dadurch gekennzeichnet, daß** das Produkt in einer enzymatisch vermittelten Reaktion zu (1R,4S)-cis-4-acetoxy-2-cyclopenten-1-ol oder zu (1S,4R)-cis-4-acetoxy-2-cyclopenten-1-ol umgesetzt wird.

## Claims

1. A process for preparing cis-cyclopent-4-ene-1,3-diol from 4-hydroxycyclopent-2-enone by selective reduction by means of complex borohydrides in the presence of trivalent rare earth metal compounds according to the following equation 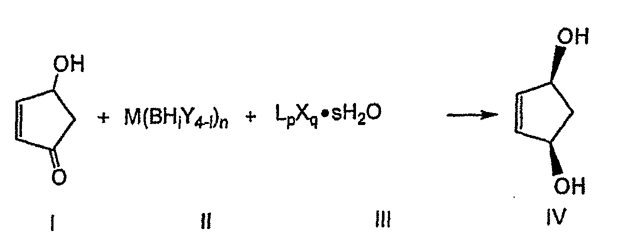 where
M is an alkali metal, an alkaline earth metal, zinc or zirconium,
i is an integer from 1 to 4,
n is an integer from 1 to 4 corresponding to the valency of M,
Y is a monovalent anion,
L is a trivalent rare earth metal (or mixtures of trivalent rare earth metals),
X is an anion of an organic or inorganic acid,
p and q are each independently integers from 1 to 4, according to the valency of X and the rare earth metal,
s is any number from 0 to 20, which represents the water content of the rare earth metal salt used, and
where the rare earth metal compound, based on the 4-hydroxycyclopent-2-enone used, is used in a substoichiometric amount.

2. A process for preparing cis-cyclopent-4-ene-1,3-diol dialkanoates from 4-hydroxycyclopent-2-enone by selective reduction by means of complex borohydrides in the presence of trivalent rare earth metal compounds followed by an acetylation with or without isolation of the cis-cyclopent-4-ene-1,3-diol formed as an intermediate according to the following equation: 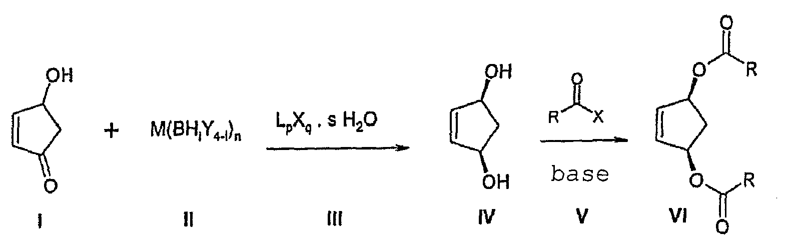 where M, i, Y, n, L, X, p, q and s are each as defined in claim 1 and
R is an aromatic or aliphatic radical and
where the rare earth metal compound, based on the 4-hydroxycyclopent-2-enone used, is used in a substoichiometric amount.

3. The process as claimed in claim 1 or 2, wherein less than 50 mol% of rare earth metal compound or mixed rare earth metal compound, based on 4-hydroxycyclopent-2-enone, is used.

4. The process as claimed in any of claims 1 to 3, wherein the trivalent rare earth metal is cerium.

5. The process as claimed in any of claims 1 to 4, wherein the trivalent rare earth metal salt is hydrated cerium(III) chloride with a water content between 1 % and 40 %.

6. The process as claimed in any of claims 1 to 4, wherein the trivalent rare earth metal salt is dry cerium(III) chloride with a water content of < 1 %.

7. The process as claimed in any of claims 1 to 6, wherein the complex borohydride is sodium borohydride, potassium borohydride, calcium borohydride or zinc borohydride.

8. The process as claimed in any of claims 1 to 7, wherein the reduction step is performed in a solvent or solvent mixture comprising methanol and optionally water, tetrahydrofuran, 2-methyltetrahydrofuran, tert-butyl methyl ether, diisopropyl ether, dipropyl ether, dibutyl ether, 1,4-dioxane, toluene, xylene, hexane, heptane or petroleum ether.

9. The process as claimed in any of claims 2 to 8, wherein the acylation step is performed in the same solvent or solvent mixture as the reduction.

10. The process as claimed in any of claims 2 to 9, wherein a solvent exchange takes place between reduction and acylation, and the acylation is performed in an aprotic solvent.

11. The process as claimed in any of claims 2 to 10, wherein acetic anhydride or acetyl chloride in the presence of a base is used for acylation.

12. The process as claimed in any of claims 2 to 11, wherein the acylating agent is obtained in situ by activating a carboxylic acid with the aid of a water-removing activating agent.

13. The process as claimed in any of claims 1 to 12, wherein the product is hydrolyzed in an enzyme-mediated reaction to give a nonracemic monoester.

14. The process as claimed in any of claims 1 to 13, wherein the product is converted in an enzyme-mediated reaction to (1R,4S)-cis-4-acetoxy-2-cyclopenten-l-ol or to (1S,4R)-cis-4-acetoxy-2-cyclopenten-1-ol.

## Revendications

1. Procédé de fabrication de cis-cyclopent-4-ène-1,3-diol à partir de 4-hydroxycyclopent-2-énone par réduction sélective au moyen d'hydrures de bore complexes en présence de composés de métaux de terres rares trivalents selon l'équation suivants : 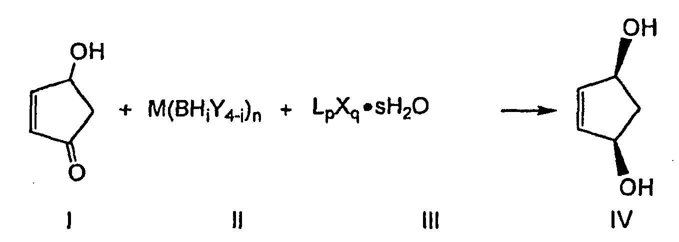 dans laquelle :
M désigne un métal alcalin, un métal alcalinoterreux, le zinc ou le zirconium,
i est un nombre entier entre 1 et 4,
n est un nombre entier entre 1 et 4 correspondant à la valence de M,
Y est un anion monovalent,
L est un métal de terre rare trivalent (ou des mélanges de métaux de terres rares trivalents),
X est un anion d'un acide organique ou inorganique,
p et q sont des nombres entiers entre 1 et 4 indépendamment l'un de l'autre correspondant à la valence de X et du métal de terre rare,
s est un nombre quelconque entre 0 et 20 qui reproduit la teneur en eau du sel de métal de terre rare utilisé, et
dans laquelle le composé de métal de terre rare est
utilisé en quantité sous-stoechiométrique par rapport à la 4-hydroxycyclopent-2-énone utilisée.

2. Procédé de fabrication de cis-cyclopent-4-ène-1,3-dioldialcanoates à partir de 4-hydroxycyclopent-2-énone par réduction sélective au moyen d'hydrures de bore complexes en présence de composés de métaux de terres rares trivalents, suivie d'une acétylation avec ou sans isolement du cis-cyclopent-4-ène-1,3-diol formé de manière intermédiaire selon l'équation suivants : 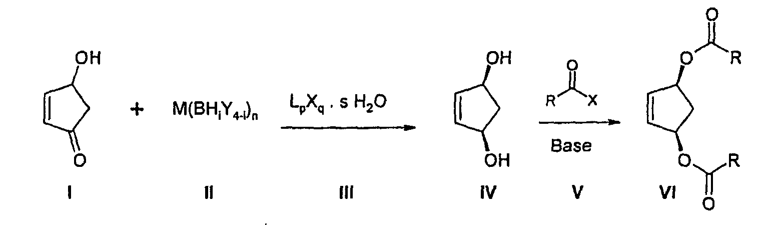 dans laquelle M, i, Y, n, L, X, p, q et s ont les
significations indiquées dans la revendication 1, et
R est un radical aromatique ou aliphatique, et
dans laquelle le composé de métal de terre rare est
utilisé en quantité sous-stoechiométrique par rapport à la 4-hydroxycyclopent-2-énone utilisée.

3. Procédé selon la revendication 1 ou la revendication 2, **caractérisé en ce qu'**on utilise moins de 50 % en mole de composé de métal de terre rare ou de mélange de composés de métaux de terres rares par rapport à la 4-hydroxycyclopent-2-énone.

4. Procédé selon l'une quelconque des revendications 1 à 3, **caractérisé en ce que** le métal de terre rare trivalent est Cer.

5. Procédé selon l'une quelconque des revendications 1 à 4, **caractérisé en ce que** le sel de métal de terre rare trivalent est le chlorure de Cer(III) hydraté avec une teneur en eau entre 1 et 40 %.

6. Procédé selon l'une quelconque des revendications 1 à 4, **caractérisé en ce que** le sel de métal de terre rare trivalent est le chlorure de Cer(III) sec avec une teneur en eau < 1 %.

7. Procédé selon l'une quelconque des revendications 1 à 6, **caractérisé en ce que** l'hydrure de bore complexe est le borohydrure de sodium, le borohydrure de potassium, le borohydrure de calcium ou le borohydrure de zinc.

8. Procédé selon l'une quelconque des revendications 1 à 7, **caractérisé en ce que** l'étape de réduction est réalisée dans un solvant ou un mélange de solvants contenant du méthanol ou éventuellement de l'eau, du tétrahydrofuranne, du 2-méthyltétrahydrofuranne de l'éther tert-butylméthylique, de l'éther diisopropylique, de l'éther dipropylique, de l'éther dibutylique, du 1,4-dioxanne, du toluène, du xylène, de l'hexane, de l'heptane ou de l'éther de pétrole.

9. Procédé selon l'une quelconque des revendications 2 à 8, **caractérisé en ce que** l'étape d'acétylation est réalisée dans le même solvant ou mélange de solvants que la réduction.

10. Procédé selon l'une quelconque des revendications 2 à 9, **caractérisé en ce que** l'on effectue un échange de solvant entre la réduction
et l'acylation et l'acylation se fait dans un solvant aprotique.

11. Procédé selon l'une quelconque des revendications 2 à 10, **caractérisé en ce que** l'on utilise pour l'acylation un anhydride d'acide acétique ou un chlorure d'acétyle en présence d'une base.

12. Procédé selon l'une quelconque des revendications 2 à 11, **caractérisé en ce que** l'agent d'acylation est produit in situ par activation d'un acide carboxylique à l'aide d'un agent d'activation déshydratant.

13. Procédé selon l'une quelconque des revendications 1 à 12, **caractérisé en ce que** le produit est saponifié par une réaction assistée par voie enzymatique pour obtenir un monoester non racémique.

14. Procédé selon l'une quelconque des revendications 1 à 13, **caractérisé en ce que** le produit est soumis à une réaction assistée par voie enzymatique pour obtenir le (1R,4S)-cis-4-acétoxy-2-cyclopentén-1-ol ou le (1S,4R)-cis-4-acétoxy-2-cyclopentén-1-ol.
